# EUROPÄISCHE PATENTSCHRIFT

(11) **EP 0 917 526 B1**
(45) Veröffentlichungstag und Bekanntmachung des Hinweises auf die Patenterteilung: **12.12.2001**
(21) Anmeldenummer: 97941915.7
(22) Anmeldetag: 06.08.1997
(51) Int. Cl.: C05G 3/08, C05G 3/00

(54) **VERWENDUNG VON POLYSÄUREN ZUR BEHANDLUNG VON MINERALDÜNGEMITTELN, DIE EINEN NITRIFIKATIONSINHIBITOR ENTHALTEN**
USE OF POLYACIDS TO TREAT MINERAL FERTILIZERS CONTAINING A NITRIFICATION INHIBITOR
UTILISATION DE POLYACIDES POUR TRAITER DES ENGRAIS MINERAUX CONTENANT UN INHIBITEUR DE NITRIFICATION

(30) Priorität: 06.08.1996 DE 19631764
(43) Veröffentlichungstag der Anmeldung: 26.05.1999
(60) Teilanmeldung: 01105436.8
(73) Patentinhaber: BASF AKTIENGESELLSCHAFT, 67056 Ludwigshafen (DE)
(72) Erfinder: BARTH, Thomas, D-67067 Ludwigshafen (DE); RIEBER, Norbert, D-68259 Mannheim (DE); GOLD, Randall, Evan, Apex, NC 27502 (US); DRESSEL, Jürgen, D-67141 Neuhofen (DE); ERHARDT, Klaus, D-69181 Leimen (DE); HORCHLER VON LOCQUENGH, Klaus, D-67117 Limburgerhof (DE); LEIBOLD, Edgar, D-67227 Frankenthal (DE); RITTINGER, Stefan, D-68199 Mannheim (DE)
(74) Vertreter: Isenbruck, Günter, Dr.
(86) Internationale Anmeldenummer: EP9704278
(87) Internationale Veröffentlichungsnummer: WO9805607

(56) Entgegenhaltungen:
- EP-A- 0 166 420
- EP-A- 0 529 473
- WO-A-89/09200
- AU-A- 5 301 279
- DD-A- 222 471
- DD-A- 273 829
- DE-A- 4 405 393
- DE-A- 4 446 194
- US-A- 3 635 690
- US-A- 4 523 940
- US-A- 4 975 107
- US-A- 5 174 806
- US-A- 5 482 529
- CHEMICAL ABSTRACTS, vol. 112, no. 13, 26.März 1990 Columbus, Ohio, US; abstract no. 117849f, F.KH. KHAZIEV ET AL.: "Composition for inhibiting nitrification of urea in soil." Seite 650; XP000181047 & SU 1 477 726 A (BASHKIR INSTITUTE OF CHEMISTRY ET AL.)
- CHEMICAL ABSTRACTS, vol. 113, no. 17, 22.Oktober 1990 Columbus, Ohio, US; abstract no. 151358a, H.J. KLASSE ET AL.: "Effect of ammonium thiosulfate on the nitrification-inhibiting activity of dicyandiamide." Seite 686; XP000182729 & VDLUFA-SCHRIFTENR., Bd. 30, 1990, Seite 181-186
- CHEMICAL ABSTRACTS, vol. 118, no. 15, 12.April 1993 Columbus, Ohio, US; abstract no. 146794p, V.D. PARKHOMENKO ET AL.: "Kinetics of the thermal decomposition of nitrification inhibitors based on 3(5)-methylpyrazole derivatives." Seite 770; XP000354133 & ZH. PRIKL. KHIM. (S.-PETERBURG), Bd. 65, Nr. 6, 1992, Seiten 1362-1367,

## Beschreibung

Die Erfindung betrifft die Verwendung von anorganischen oder organischen Polysäuren zur Behandlung von Mineraldüngemitteln.

Insbesondere betrifft die Erfindung die Verwendung der Polysäuren als Gemisch mit mindestens einem Nitrifikationsinhibitor zur Behandlung von Mineraldüngemitteln, sowie die entsprechenden behandelten Mineraldüngemittel an sich.

Um Pflanzen in der Landwirtschaft den von ihnen benötigten Stickstoff zur Verfügung zu stellen, werden im wesentlichen Ammoniumverbindungen als Düngemittel eingesetzt.

Ammoniumverbindungen werden im Boden in relativ kurzer Zeit mikrobiell zu Nitrat umgesetzt (Nitrifikation). Nitrat kann jedoch aus dem Boden ausgewaschen werden. Der ausgewaschene Anteil steht dabei für die Pflanzenernährung nicht mehr zur Verfügung, so daß aus diesem Grund eine schnelle Nitrifikation unerwünscht ist. Zur besseren Ausnutzung des Düngers werden deshalb dem Dünger Nitrifikationsinhibitoren zugesetzt. Eine bekannte Gruppe von Nitrifikationsinhibitoren sind Pyrazolverbindungen.

Ein Problem bei der Verwendung von Pyrazolverbindungen als Nitrifikationsinhibitoren ist deren hohe Flüchtigkeit. Bei der Lagerung von Pyrazolverbindungen enthaltenden Düngerzubereitungen tritt somit ein kontinuierlicher Verlust an Wirkstoff durch Verdampfung auf. Deshalb müssen die Pyrazolverbindungen durch geeignete Maßnahmen in einer nichtflüchtigen Form formuliert werden.

Zur Fixierung der Pyrazolverbindungen wurden diese beispielsweise in Übergangsmetallkomplexe wie Zinkkomplexe überführt. Dies ist beispielsweise in der US 4 522 642 beschrieben. Die Flüchtigkeit der Wirkstoffe läßt sich damit herabsetzen. Aus Umweltschutzgründen ist die großflächige Ausbringung von Zink, Kupfer oder Mangan auf den Erdboden jedoch unerwünscht. Komplexe von Alkali- oder Erdalkalimetallen, die umweltverträglich sind, sind jedoch nicht ausreichend stabil und hydrolysieren in wäßriger Umgebung.

Weiterhin wurde versucht, durch Neutralisierung der Pyrazolverbindungen mit Mineralsäuren, wie Phosphorsäure oder Salzsäure, ihre Flüchtigkeit zu vermindem. In der DE-A-4 128 828 ist die Verwendung von Nitraten und Phosphaten des 3-Methylpyrazols zur Beschichtung von Düngern beschrieben. Die US 3 635 690 beschreibt ebenfalls die Stabilisierung von Pyrazolderivaten durch Mineralsäuren, wie Salzsäure, Schwefelsäure, Salpetersäure und Phosphorsäure. Diese sauren Salze der Pyrazolverbindungen sind jedoch immer noch hydrolyseanfällig und aus diesem Grund nicht für alle Anwendungen einsetzbar.

In der DE-A-4 128 828 wird ferner die Versiegelung des beschichteten Düngemittels mit Wachs oder Öl beschrieben. Dieses Verfahren führt bei hygroskopischen Wirkstoffsalzen jedoch nicht zu einer zufriedenstellenden Hydrolysebeständigkeit.

Ferner wurden Formulierungen von Pyrazolen mit polymeren Hilfsstoffen eingesetzt. So sind in der DD 260 486 Formulierungen von Pyrazolen in Hamstoff-Formaldehyd-Kondensaten beschrieben. Der Einbau der Wirkstoffe in die Polymermatrix unterbindet jedoch die Mobilität der Wirkstoffe im Erdboden. Deshalb müssen bei dieser Anwendungsform die feinverteilte Formulierung und Dünger mit dem zu düngenden Boden gründlich durchmischt werden. Andernfalls verbleibt der Nitrifikationsinhibitor mit der Polymermatrix an der Erdoberfläche. Die Notwendigkeit des Durchmischens von Formulierung, Düngemittel und Erdboden ist jedoch aufwendig.

Aufgabe der vorliegenden Erfindung ist die Bereitstellung von Mineraldüngemitteln, die einen Nitrifikationsinhibitor enthalten, dessen Gehalt sich bei Lagerung und Ausbringung des Düngers nicht wesentlich verändert, der nach der Ausbringung des Düngers im Erdreich verbleibt und seine Wirkung dort entfalten kann.

Gelöst wird diese Aufgabe durch Verwendung von anorganischen oder organischen Polysäuren zur Behandlung von Mineraldüngemitteln in Pulverform oder in Granulatform. Dabei enthält das behandelte Mineraldüngemittel einen Nitrifikationsinhibitor, der im Mineraldüngemittel oder auf dessen Oberfläche vorliegt. Ferner kann der Nitrifikationsinhibitor auch als Gemisch mit der Polysäure eingesetzt werden und gelangt dann bei der Behandlung des erfindungsgemäß eingesetzten Mineraldüngers in diesen, vorzugsweise auf die Oberfläche desselben.

Die Verwendung von anorganischen oder organischen Polysäuren zur Behandlung von Mineraldüngemitteln, die Nitrifikationsinhibitoren enthalten, führt zu einer verbesserten Fixierung der Nitrifikationsinhibitoren im Mineraldüngemittel. Die Flüchtigkeit des Nitrifikationsinhibitors wird dabei stark herabgesetzt, so daß die Lagerstabilität des behandelten Mineraldüngemittels steigt. Ein Verlust an Nitrifikationsinhibitor während einer Lagerzeit oder beim Ausbringen auf den Boden wird vermieden.

Zudem weisen die erfindungsgemäße Behandlung und die so erhaltenen behandelten Mineraldüngemittel den Vorteil der ökologischen Unbedenklichkeit auf. Sie enthalten keine toxischen Substanzen, wie beispielsweise Zink, Kupfer oder Mangan, die in größeren Mengen die Umweltverträglichkeit sehr stark einschränken und zur Kontaminierung von Böden führen können.

Weiterhin kann die erfindungsgemäße Behandlung kostengünstig und ökologisch verträglich durchgeführt werden. Durch die erfindungsgemäße Behandlung kann wegen der verminderten Flüchtigkeit die Menge an Nitrifikationsinhibitoren im Mineraldüngemittel herabgesetzt werden, was zu verringerten Kosten und zu einer besseren Umweltverträglichkeit der erfindungsgemäßen Düngemittel führt.

### Polysäuren

Erfindungsgemäß werden anorganische oder organische Polysäuren zur Behandlung der Mineraldüngemittel verwendet.

Dabei können alle geeigneten anorganischen oder organischen Polysäuren verwendet werden, die die Verdampfungstendenz von Nitrifikationsinhibitoren vermindern.

Als anorganische Polysäuren können erfindungsgemäß Isopolysäuren oder Heteropolysäuren verwendet werden, insbesondere Polyphosphorsäuren oder Polykieselsäuren. Die Polyphosphorsäuren weisen zum Beispiel die allgemeine Formel Hₙ₊₂PₙO₃ₙ₊₁ auf, wobei n eine ganze Zahl von mindestens 2, vorzugsweise mindestens 10 ist.

Weitere verwendbare anorganische Polysäuren sind dem Fachmann bekannt.

Als organische Polysäuren kommen solche Polymere in Betracht, die eine Mehrzahl von freien Carbonsäuregruppen aufweisen. Dabei kann es sich um Homo- oder Copolymere handeln. Als carboxylgruppenhaltige bzw. carbonsäuregruppenhaltige Monomere kommen insbesondere monoethylenisch ungesättigte Mono- oder Dicarbonsäuren mit 3 bis 6 C-Atomen oder deren entsprechende Anhydride in Betracht, wie beispielsweise Acrylsäure, Methacrylsäure, Ethylacrylsäure, Allylessigsäure, Crotonsäure, Vinylessigsäure, Maleinsäure, Itaconsäure, Mesaconsäure, Furmarsäure, Citraconsäure, Methylenmalonsäure, sowie deren Ester, wie z.B. Maleinsäuremonoalkylester, und Gemische davon. Im Falle von Dicarbonsäuremonoalkylestern bezieht sich die angegebene Anzahl an C-Atomen auf das Dicarbonsäuregerüst, die Alkylgruppe im Esterrest kann davon unabhängig 1 bis 20 C-Atome, insbesondere 1 bis 8 C-Atome aufweisen. Als entsprechende monoethylenisch ungesättigte Dicarbonsäureanhydride kommen Maleinsäureanhydrid, Itaconsäureanhydrid, Citraconsäureanhydrid und deren Gemische in Betracht. Bevorzugt werden Acrylsäure, Methacrylsäure, Maleinsäure, Itaconsäure und Maleinsäureanhydrid eingesetzt. Besonders bevorzugt wird Acrylsäure eingesetzt.

Diese Carboxylgruppen enthaltenden oder Carbonsäuregruppen enthaltenden Monomere können homopolymerisiert oder copolymerisiert werden mit weiteren vinylischen Monomeren, wie beispielsweise C₁₋₈-, vorzugsweise C₁₋₄-Alkylenen, insbesondere Ethylen oder Propylen.

Besonders bevorzugt wird als organische Polysäure Polyacrylsäure oder Polymethacrylsäure verwendet.

Die anorganischen oder organischen Polysäuren können als freie Säuren oder als teilweise neutralisierte Ammonium-, Alkali- oder Erdalkalimetallsalze davon eingesetzt werden. Vorzugsweise werden sie als freie Säuren eingesetzt.

Insbesondere bevorzugt sind Polyphosphorsäure und Poly(meth)acrylsäure.

Das mittlere Molekulargewicht der organischen Polysäuren beträgt vorzugsweise 10.000 bis 500.000, besonders bevorzugt 10.000 bis 100.000, insbesondere 30.000 bis 70.000.

Verfahren zur Herstellung der Polysäuren sind dem Fachmann bekannt.

### Nitrifikationsinhibitor

In den erfindungsgemäßen Mineraldüngemitteln können beliebige geeignete Nitrifikationsinhibitoren eingesetzt werden.

Besonders vorteilhaft werden die erfindungsgemäß verwendeten Polysäuren zur Behandlung von Mineraldüngemitteln, die diese flüchtigen Nitrifikationsinhibitoren, insbesondere Pyrazolverbindungen, enthalten, eingesetzt. Unter "Pyrazolverbindungen" werden alle Pyrazolverbindungen verstanden, die eine nitrifikationsinhibierende Wirkung aufweisen, wie sie z.B. auch in den eingangs bei der Diskussion des Standes der Technik erwähnten Druckschriften US 3 635 690, US 4 522 642 und DE-A-4 128 828, deren Inhalt bezüglich der dort beschriebenen Pyrazolverbindungen hier einbezogen wird, beschrieben sind.

Gemäß einer Ausführungsform handelt es sich bei den als Nitrifikationsinhibitoren eingesetzten Pyrazolverbindungen um Verbindungen der nachstehenden allgemeinen Formel 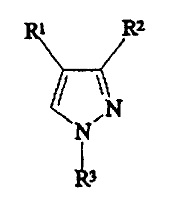 wobei die Reste R¹, R² und R³ unabhängig voneinander Halogenatome, Nitrogruppen, Wasserstoffatome oder C₁₋₂₀-, vorzugsweise C₁₋₄-Alkylreste, C₃₋₈-Cycloalkylreste, C₅₋₂₀-Arylreste oder Alkylarylreste sind, wobei die letztgenannten 4 Reste einfach oder dreifach durch Halogenatome und/oder Hydroxylgruppen substituiert sein können.

Vorzugsweise ist der Rest R¹ ein Wasserstoffatom, ein Halogenatom, oder ein C₁₋₄-Alkylrest, der Rest R² ein C₁₋₄-Alkylrest und der Rest R³ ein Wasserstoffatom oder ein Rest -CH₂OH.

Gemäß einer weiteren Ausführungsform der Erfindung ist in der vorstehenden Formel der Rest R¹ ein Halogenatom oder C₁₋₄-Alkylrest, der Rest R² ein C₁₋₄-Alkylrest und der Rest R³ ein Wasserstoffatom oder ein Rest -CH₂CH₂COOH oder -CH₂CH(CH₃)COOH.

Die Pyrazolverbindungen können in der basischen Form eingesetzt werden, wie auch in Form von Säureadditionssalzen mit anorganischen Mineralsäuren und organischen Säuren. Beispiele anorganischer Mineralsäuren sind Salzsäure, Phosphorsäure, Schwefelsäure, vorzugsweise Phosphorsäure. Beispiele organischer Säuren sind Ameisensäure, Essigsäure, wie auch Fettsäuren.
Beispiele entsprechender Salze sind die Hydrochloride und Phosphorsäure-Additionssalze.

Die Pyrazolverbindungen können einzeln oder in Form von Gemischen eingesetzt werden.

Besonders bevorzugte Pyrazolverbindungen sind 3,4-Dimethylpyrazol, 4-Chlor-3-methylpyrazol, N-Hydroxymethyl-3,4-dimethylpyrazol, N-Hydroxymethyl-4-chlor-3-methylpyrazol sowie die Phosphorsäure-Additionssalze von 3,4-Dimethylpyrazol und 4-Chlor-3-methylpyrazol wie auch das Hydrochlorid von 3,4-Dimethylpyrazol.

Durch Verwendung der Säureadditionssalze der Pyrazolverbindungen kann die Flüchtigkeit der Verbindungen weiter herabgesetzt werden. Somit werden vorteilhaft Säureadditionssalze der Pyrazolverbindungen in Kombination mit der erfindungsgemäßen Behandlung eingesetzt.

Als Halogenatome werden in den vorstehenden Verbindungen Fluor, Chlor, Brom oder Iod, vorzugsweise Fluor, Chlor oder Brom eingesetzt.

Die Herstellung der erfindungsgemäß verwendeten Pyrawlverbindungen ist bekannt. Sie ist beispielsweise beschrieben in EP-A-0 474 037, DE-A 3 840 342, EP-A-0 467 707. Zur Herstellung der N-Hydroxymethylpyrazole werden die entsprechenden Pyrazole in Methanol mit Formalinlösung umgesetzt. Überschüssiges Lösungsmittel wird sodann verdampft, wobei die Verbindungen als Festkörper anfallen. Zur Herstellung von 3,4-Dimethylpyrazol kann zudem auf Noyce et al., Jour. of Org. Chem. 20, 1955, 1681 bis 1682 verwiesen werden.

Die Säureadditionssalze der Pyrazolverbindungen erhält man durch Umsetzung der Pyrazole mit einem Äquivalent an entsprechender Säure. Die Herstellung des Hydrochlorids von 4-Chlor-3-methylpyrazol ist in Hüttel et al., Liebigs Anm. Chem. 1956, 598, 186, 194 beschrieben.

### Behandelte Mineraldüngemittel

Erfindungsgemäß werden Mineraldüngemittel eingesetzt. Dies sind Ammonium oder Harnstoff enthaltende Düngemittel. Beispiele derartiger ammoniumhaltiger Düngemittel sind NPK-Düngemittel, Kalkammonsalpeter, Ammonsulfatsalpeter, Ammoniumsulfat oder Arnmoniumphosphat.

Die erfindungsgemäß behandelten Mineraldüngemittel liegen in Pulverform oder in Granulatform vor.

Das erfindungsgemäße behandelte Mineraldüngemittel enthält mindestens einen Nitrifikationsinhibitor und wird mit mindestens einer anorganischen oder organischen Polysäure behandelt. Dabei kann das Mineraldüngemittel im Gemisch mit dem Nitrifikationsinhibitor vorliegen. Der Nitrifikationsinhibitor kann auch auf der Oberfläche des Mineraldüngemittels vorliegen und sodann mit der erfindungsgemäßen Polysäure behandelt werden. Vorzugsweise kann der Nitrifikationsinhibitor im Gemisch mit der erfindungsgemäß verwendeten Polysäure auf das Mineraldüngemittel aufgebracht werden.

Vorzugsweise enthält das Mineraldüngemittel als Nitrifikationsinhibitor eine Pyrazolverbindung oder ein Säuresalz davon. Vorzugsweise wird das das Mineraldüngemittel mit Poly(meth)acrylsäure oder Polyphosphorsäure behandelt.

Vorzugsweise enthält das Mineraldüngemittel 0,01 bis 1,5 Gew.-% an Nitrifikationsinhibitor und 0,01 bis 1,5 Gew.-% an Polysäure, bezogen auf das behandelte Mineraldüngemittel.

Ferner betrifft die vorliegende Erfindung ein behandeltes Mineraldüngemittel, enthaltend das vorstehend aufgeführte Mineraldüngemittel, das mit mindestens einer anorganischen oder organischen Polysäure oder einem Gemisch aus mindestens einem Nitrifikationsinhibitor und mindestens einer anorganischen oder organischen Polysäure behandelt ist.

### Herstellung der behandelten Mineraldüngemittel

Die erfindungsgemäßen behandelten Mineraldüngemittel werden hergestellt, indem man das Mineraldüngemittel mit der Polysäure behandelt. Vorzugsweise werden sie so hergestellt, daß man die Oberfläche des Mineraldüngemittels mit der Polysäure behandelt.

Dabei kann der Nitrifikationsinhibitor im Gemisch mit dem Mineraldüngemittel vorliegen oder vor der Polysäure auf das Mineraldüngemittel aufgebracht worden sein. Gemäß einer Ausführungsform der Erfindung wird das Mineraldüngemittel mit Polysäure und Nitrifikationsinhibitor als Gemisch behandelt.

Dabei werden die Mineraldüngemittel mit der Polysäure, dem Nitrifikationsinhibitor oder deren Gemisch behandelt, z.B. imprägniert oder besprüht, indem man sie mit einer flüssigen Zubereitung, z.B. einer Lösung oder Suspension der Polysäure, des Nitrifikationsinhibitors oder des Gemisches besprüht und gegebenenfalls wieder trocknet. Ein entsprechendes Verfahren ist beispielsweise beschrieben in der DE-A-4 128 828.

Vorzugsweise werden Nitrifikationsinhibitor und Polysäure in Form einer flüssigen Zubereitung, z.B. einer Lösung oder Suspension der Polysäure auf das Mineraldüngemittel aufgebracht, z.B. gesprüht, und gegebenenfalls anschließend getrocknet.

Dabei wird vorzugsweise ein Gemisch eingesetzt aus 30 bis 98 Gew.-% mindestens einer Polysäure und 2 bis 70 Gew.-% mindestens eines Nitrifikationsinhibitors in einem flüssigen Medium, vorzugsweise Wasser. Der Nitrifikationsinhibitor kann dabei wiederum als Säureadditionssalz vorliegen.

Ferner betrifft die vorliegende Erfindung ein Düngeverfahren, wobei man behandelte Mineraldüngemittel, wie sie im Rahmen der vorliegenden Anmeldung definiert sind, auf den Ackerboden ausbringt.

Dabei werden die beschriebenen Mineraldüngemittel gemäß einer Ausführungsform der Erfindung nicht zur Behandlung von Böden, die für Mais-, Baumwolle-, Weizen-, Reis-, Gerste- und/oder Zuckerrübenkulturen vorgesehen sind, oder der entsprechenden Kulturen eingesetzt.

Nachstehend wird die Erfindung anhand von Beispielen näher beschrieben.

### Beispiele

### Herstellung von N-Hydroxymethyl-3,4-dimethylpyrazol

96 g (1,0 mol) 3,4-Dimethylpyrazol in 50 ml Methanol wurden in 100 g (1,0 mol) Formalin-Lösung (30%) bei Raumtemperatur gelöst. Sodann wurden Wasser und Methanol abgedampft. Die Titelverbindung blieb als weißer Feststoff zurück (Ausbeute 95%).

### Herstellung von N-Hydroxymethyl-4-chlor-3-methylpyrazol

116 g (1,0 mol) 4-Chlor-3-methylpyrazol in 50 ml Methanol wurden in 100 g (1,0 mol) Formalin-Lösung (30%) bei Raumtemperatur gelöst. Sodann wurden Wasser und Methanol abgedampft. Die Titelverbindung blieb als weißer Feststoff zurück (Ausbeute 95%).

### Herstellung von 3,4-Dimethylpyrazoliumdihydrogenphosphat

96 g (1,0 mol) 3,4-Dimethylpyrazol wurden in 115 g (1,0 mol) Phosphorsäure (85 %) bei Raumtemperatur gelöst. Das in der Phosphorsäure enthaltene Wasser wurde abgedampft. Nach einigen Stunden kristallisierte die Titelverbindung aus dem zunächst vorliegenden Öl (Ausbeute 98 %).

### Herstellung von 4-Chlor-3-methylpyrazoliumdihydrogenphosphat

116 g (1,0 mol) 4-Chlor-3-methylpyrazol wurden in 115 g (1,0 mol) Phosphorsäure (85%) bei Raumtemperatur gelöst. Das in der Phosphorsäure enthaltene Wasser wurde abgedampft. Nach einigen Stunden kristallisierte die Titelverbindung aus dem zunächst vorliegenden Öl (Ausbeute 98 %).

### Herstellung der nitrifikationsinhibierten Mineraldüngemittel

Als Trägerdünger diente Ammonsulfatsalpeter (ASS). 2 g Pyrazol wurden in wenig Wasser gelöst, gegebenenfalls (s. Tabelle I) mit einer stöchiometrischen Menge Phosphorsäure (1:1) versetzt und mit 1 bis 10 g Polyacrylsäure oder Polyphosphorsäure versetzt. 2 kg des Trägerdüngers in Form eines Granulats wurden auf etwa 50°C vorgewärmt und auf einem Drehteller mit dem Gemisch, das die Pyrazolverbindung enthält, langsam besprüht. Zur Beschleunigung der Trocknung wurde mit heißer Luft nachgetrocknet, entweder am Ende des Besprühens oder nach einer Unterbrechung des Besprühens.

### Untersuchung der Lagerstabilität

Die Lagerstabilität der behandelten Mineraldüngemittel wurde in einem Schnelltest bestimmt, bei dem die nitrifikationsinhibierten Mineraldüngemittel für 4 Wochen bei 30°C, 40 bis 50% relativer Luftfeuchtigkeit und etwa 1,2 m/s Luftgeschwindigkeit in einem belüfteten Wärmeschrank gelagert wurden. Die Nitrifikationsinhibitorkonzentration auf dem Mineraldüngemittel wurde vor und nach der Lagerung bestimmt und der Verlust an Nitrifikationsinhibitor in Prozent ermittelt. Es wurden dabei jeweils etwa 10 bis 30 g behandeltes Mineraldüngemittel gelagert. Die Konzentration an Pyrazolverbindung als Nitrifikationsinhibitor betrug dabei zu Beginn der Untersuchung 0,05 Gew.-% bis 0,2 Gew.-%, bezogen auf das behandelte Mineraldüngemittel. Die für unterschiedliche Pyrazolverbindungen erhaltenen Verluste sind in der nachstehenden Tabelle I aufgeführt.

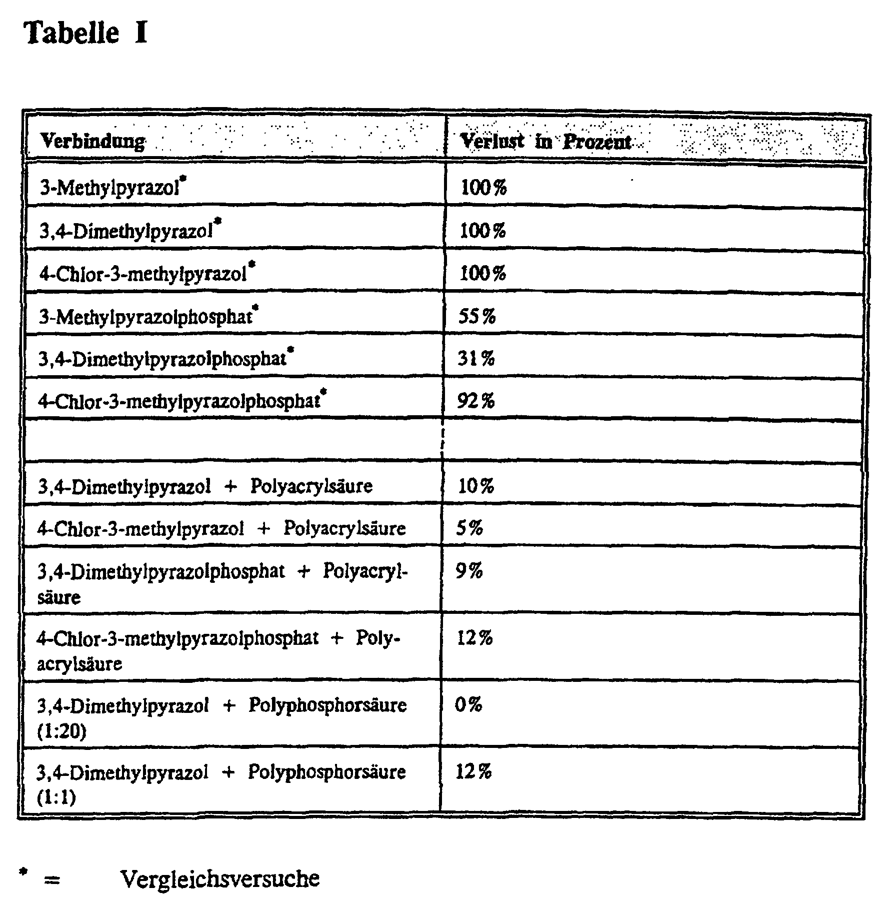

Aus den Ergebnissen aus Tabelle 1 geht hervor, daß der Verlust an Pyrazolverbindung in den erfindungsgemäßen behandeldeten Mineraldüngemittel bei der Lagerung wesentlich geringer ist als bei den Vergleichssubstanzen. Die Beschichtung mit der erfindungsgemäßen Polysäure führt zu einem wesentlich verminderten Verlust an Nitrifikationsinhibitor.

### Nachweis der biologischen Wirkung der Nitrifikationsinhibitoren - Feldversuche -

Die biologische Wirksamkeit von 4 Chlor-3-methylpyrazol (4Cl-3MP) und 3,4-Dimethylpyrazol (3,4-DMP) im Vergleich zu DCD und der Kontrolle wurde in mehreren Feldversuchen in unterschiedlichen Umwelten an Hand der Merkmale "Nitratgehalt in der Stengelbasis", "NO₃- und NH₄-N-Gehalt im Boden" sowie "Kornertrag" geprüft.

Für die Anlage, Beprobung, Beerntung und Auswertung der Feldversuche sind die im landwirtschaftlichen Versuchswesen üblichen Verfahren angewendet worden.

Die Analyse der Pflanzen- und Bodenproben erfolgte nach Standardmethoden. Die übrigen produktionstechnischen Maßnahmen, wie der Pflanzenschutz, entsprachen der guten landwirtschaftlichen Praxis und wurden einheitlich durchgeführt.

Ein biologisch wirksamer Nitrifikationsinhibitor zeichnet sich vorzugsweise dadurch aus, daß er in einem Zeitraum von bis zu 8 Wochen nach der Applikation gegenüber der Kontrolle (hier Trägerdünger Ammonsulfatsalpeter ohne Nitrifikationshemmstoff) im Boden niedrigere Gehalte an NO₃-N und höhere Gehalte an NH₄-N aufweist (Tab. 1). Als Folge dieser Bedingungen ist die Nitrataufnahme von Pflanzen reduziert (vgl. NO₃-Gehalt in der Stengelbasis von Rapspflanzen, Tab. 2) und häufig der Ertrag erhöht (vgl. Kornertrag von Winterweizen, Tab. 3a und 3b). Tabelle 4 gibt die Standortbeschreibung der Feldversuche wieder.

In der folgenden Tabelle 1 sind die Ergebnisse zusammengefaßt. Es wird deutlich, daß alle drei Nitrifikationsinhibitoren eine gute biologische Wirksamkeit im Vergleich zur Kontrolle aufweisen. 4Cl-3MP und 3,4-DMP zeichnen sich durch eine ebenso gute und z. T. bessere Wirksamkeit gegenüber DCD aus, bei allerdings erheblich niedrigeren Wirkstoffmengen. Weitere Ergebnisse zu ausgewählten Nitrifikationsinhibitoren sind in Tabelle 5 dargestellt.

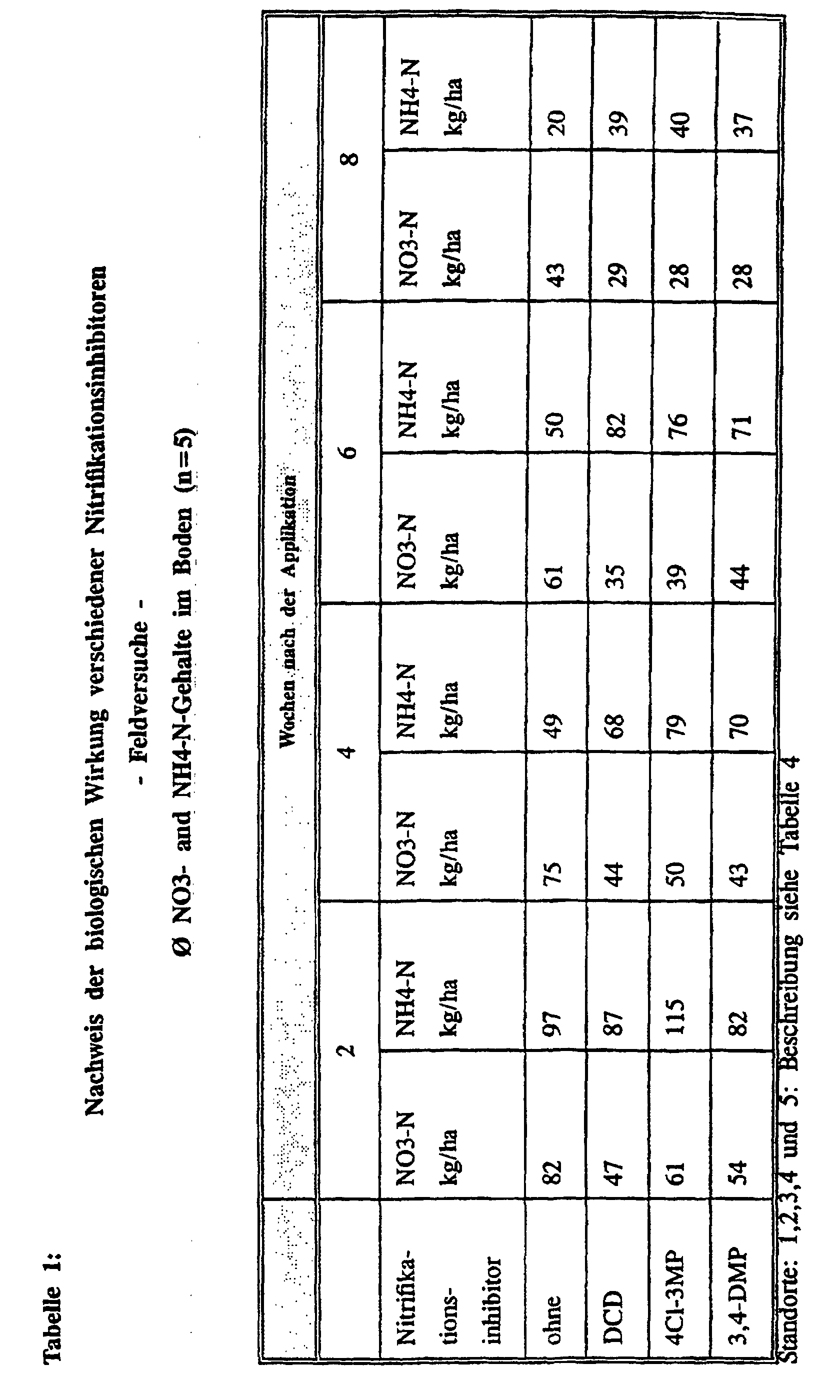

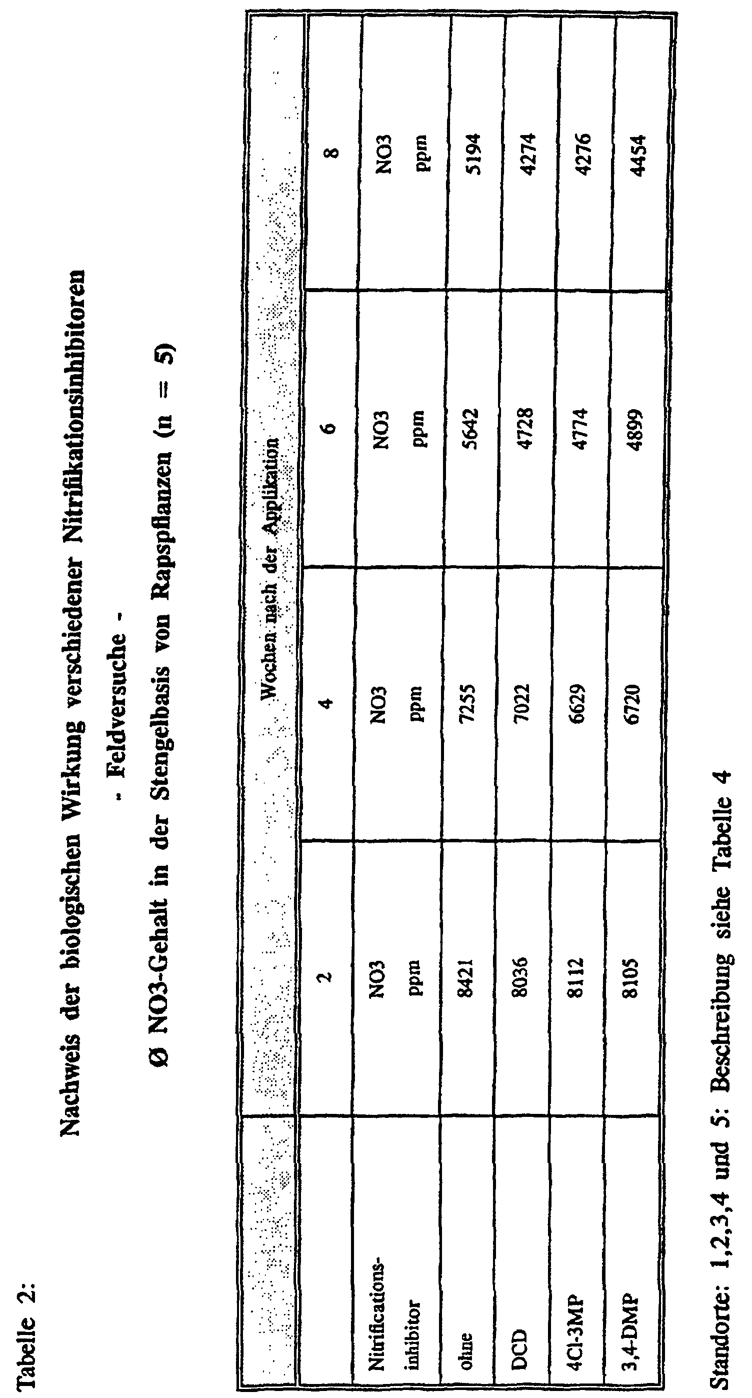

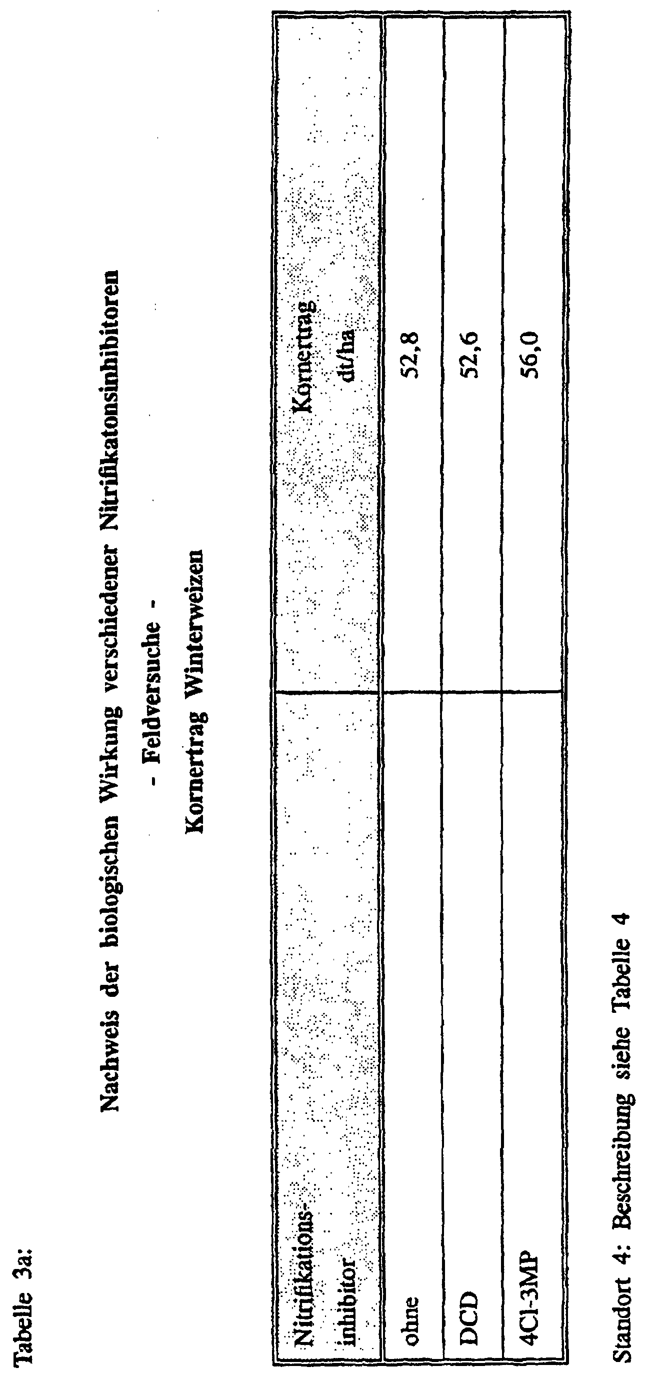

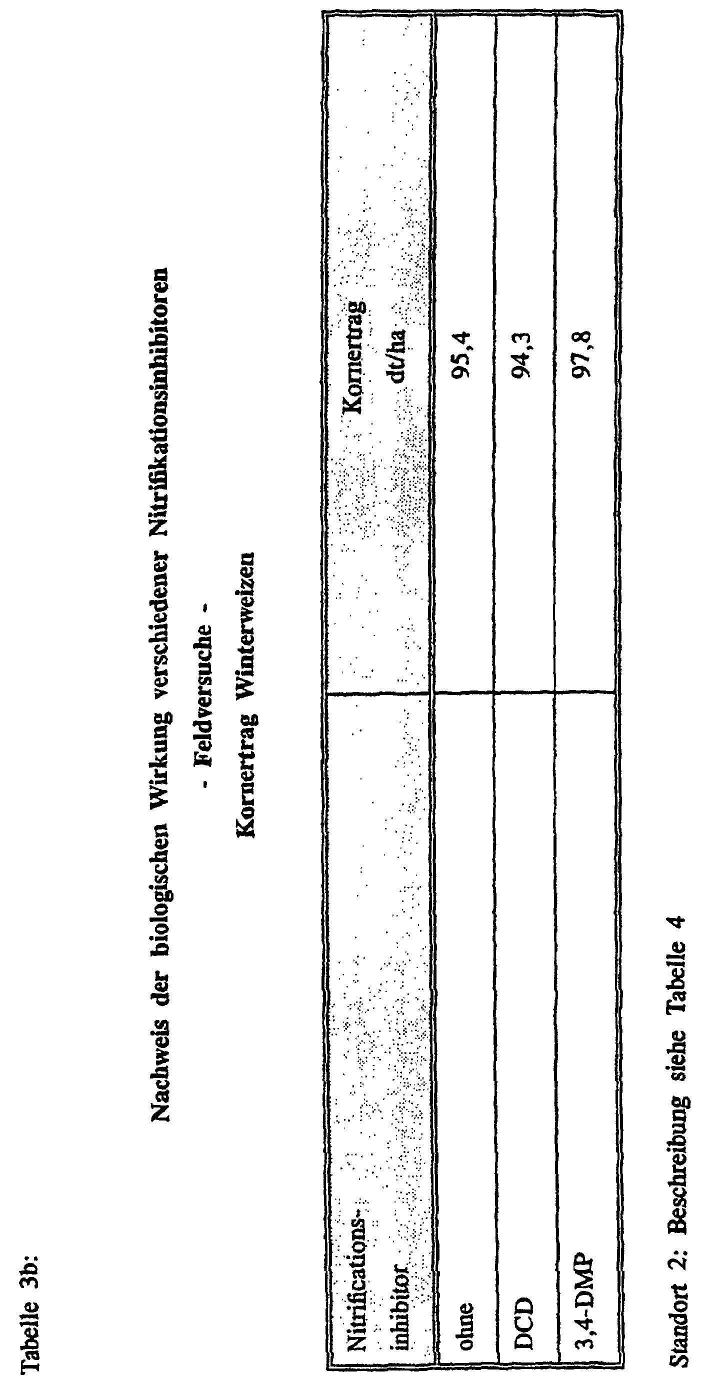

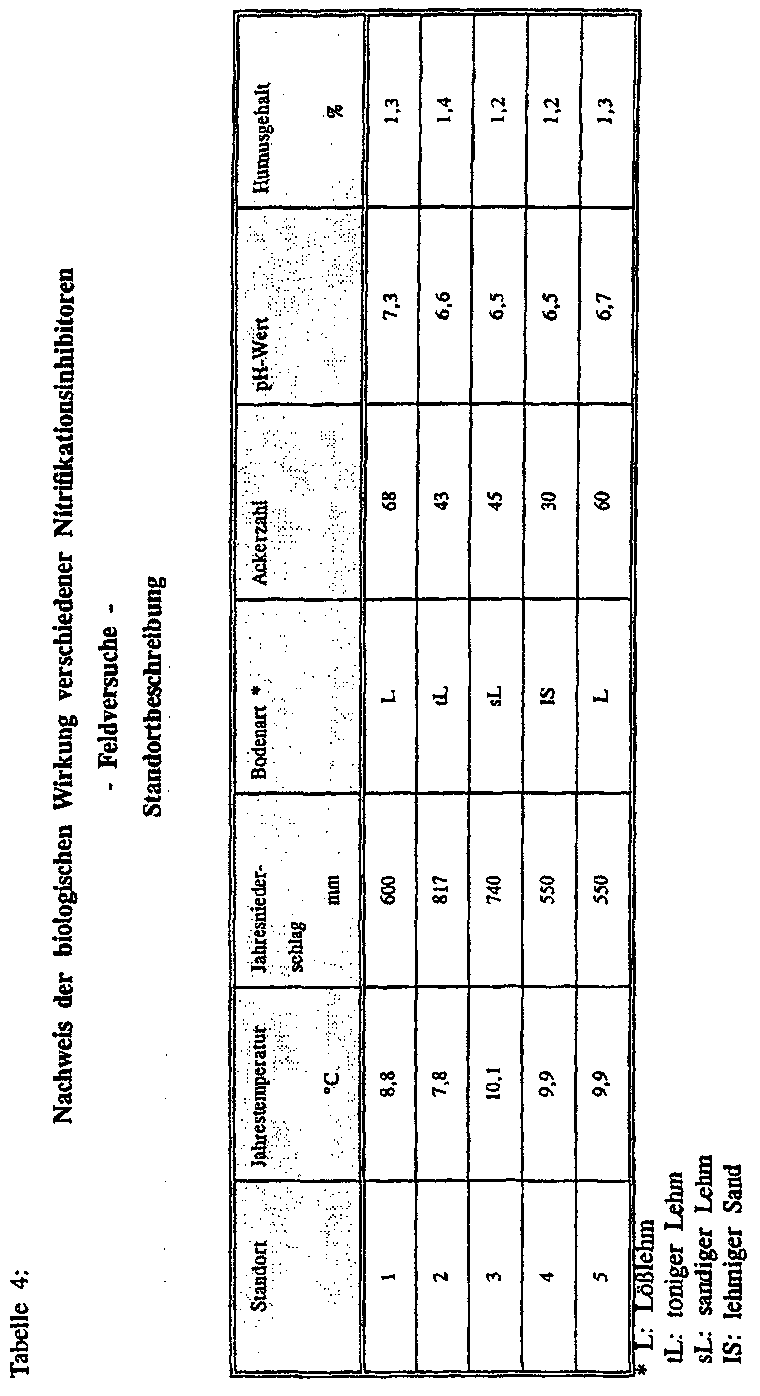

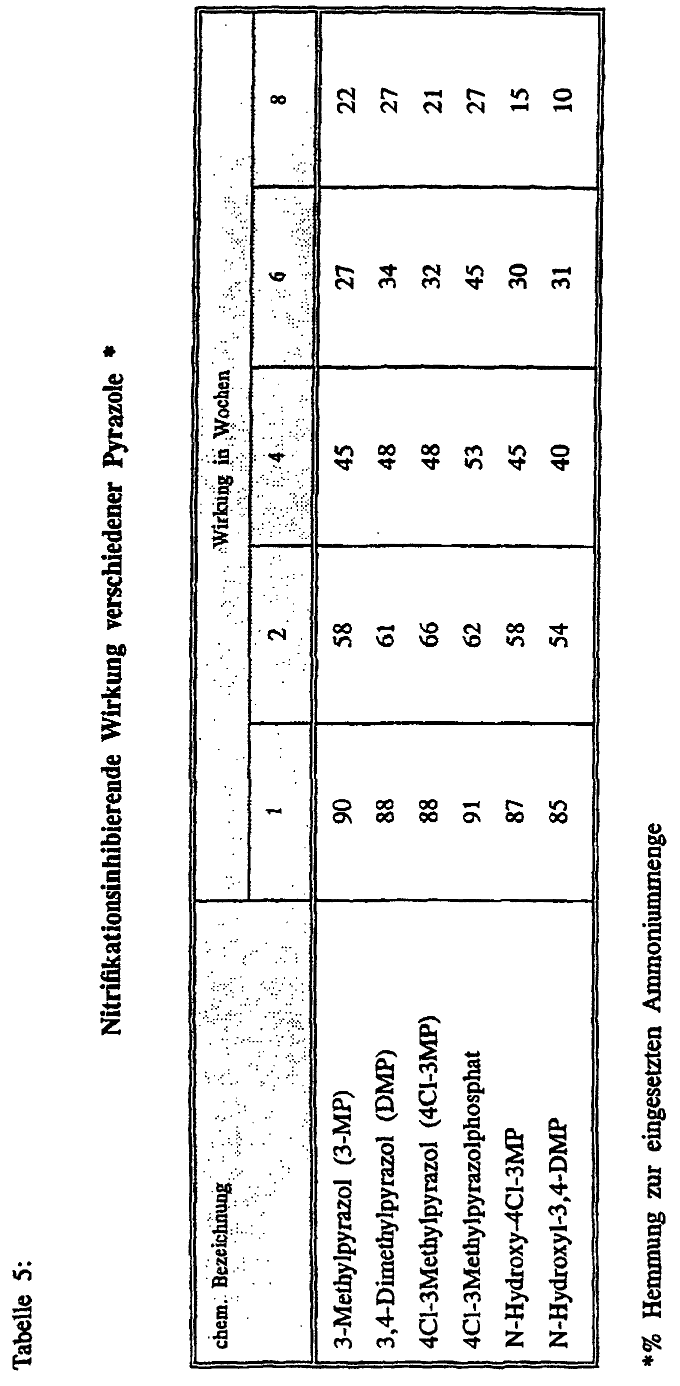

## Patentansprüche

1. Verwendung von anorganischen oder organischen Polysäuren zur Behandlung von Mineraldüngemitteln in Pulverform oder in Granulatform die einen Nitrifikationsinhibitor enthalten.

2. Verwendung nach Anspruch 1, **dadurch gekennzeichnet, daß** die Polysäuren als Gemisch mit mindestens einem Nitrifikationsinhibitor eingesetzt werden.

3. Einen Nitrifikationsinhibitor enthaltendes behandeltes Mineraldüngemittel in Pulverform oder in Granulatform, behandelt mit mindestens einer anorganischen oder organischen Polysäure oder einem Gemisch aus mindestens einem Nitrifikationsinhibitor und mindestens einer anorganischen oder organischen Polysäure.

4. Behandeltes Mineraldüngemittel nach Anspruch 3, **dadurch gekennzeichnet, daß** die Polysäure in einer Menge von 0,01 bis 1,5 Gew.-% und der Nitrifikationsinhibitor in einer Menge von 0,01 bis 1,5 Gew.-%, bezogen auf das behandelte Mineraldüngemittel, vorliegt.

5. Verfahren zur Herstellung eines behandelten Mineraldüngemittels nach Anspruch 3, **dadurch gekennzeichnet, daß** man das Düngemittel mit mindestens einer anorganischen oder organischen Polysäure oder einem Gemisch aus mindestens einem Nitrifikationsinhibitor und mindestens einer anorganischen oder organischen Polysäure behandelt.

6. Düngeverfahren, **dadurch gekennzeichnet, daß** man Mineraldüngemittel, wie sie in einem der Ansprüche 3 oder 4 definiert sind, auf Ackerboden ausbringt.

## Claims

1. The use of inorganic or organic polyacids for the treatment of inorganic fertilizers in powder form or in granule form which contain a nitrification inhibitor.

2. The use as claimed in Claim 1, wherein the polyacids are employed as a mixture with at least one nitrification inhibitor.

3. A nitrification inhibitor-containing treated inorganic fertilizer in powder form or in granule form, treated with at least one inorganic or organic polyacid or a mixture of at least one nitrification inhibitor and at least one inorganic or organic polyacid.

4. A treated inorganic fertilizer as claimed in Claim 3, wherein the polyacid is present in an amount from 0.01 to 1.5% by weight and the nitrification inhibitor in an amount from 0.01 to 1.5% by weight, based on the treated inorganic fertilizer.

5. A process for the preparation of a treated inorganic fertilizer as claimed in Claim 3, which comprises treating the fertilizer with at least one inorganic or organic polyacid or a mixture of at least one nitrification inhibitor and at least one inorganic or organic polyacid.

6. A fertilization process, wherein inorganic fertilizer, as defined in either of Claims 3 or 4, is applied to arable soil.

## Revendications

1. Utilisation de polyacides inorganiques ou organiques pour le traitement d'engrais minéraux, sous forme de poudres ou de granulés, contenant un inhibiteur de nitrification.

2. Utilisation selon la revendication 1, **caractérisée en ce que** les polyacides sont mis en oeuvre en mélange avec au moins un inhibiteur de nitrification.

3. Engrais minéral traité, sous forme de poudre ou de granulés, contenant un inhibiteur de nitrification, traité par au moins un polyacide inorganique ou organique ou par un mélange d'au moins un inhibiteur de nitrification et d'au moins un polyacide inorganique ou organique.

4. Engrais minéral traité selon la revendication 3, **caractérisé en ce que** les polyacides sont présents en quantités de 0,01 à 1,5% en poids, et l'inhibiteur de nitrification en quantités de 0,01 à 1,5% en poids, par rapport à l'engrais minéral traité.

5. Procédé de préparation d'un engrais minéral traité selon la revendication 3, **caractérisé en ce que** l'on traite l'engrais par au moins un polyacide inorganique ou organique ou par un mélange d'au moins un inhibiteur de nitrification et d'au moins un polyacide inorganique ou organique.

6. Procédé de fertilisation **caractérisé en ce que** l'on épand sur un sol agricole des engrais minéraux tels que définis dans l'une des revendications 3 ou 4.
